# EUROPEAN PATENT APPLICATION

(11) **EP 2 194 050 A1**
(43) Date of publication of application: **09.06.2010**
(21) Application number: 09158339.3
(22) Date of filing: 21.04.2009
(51) Int. Cl.: C07D 403/10, C07D 257/04, C07C 253/30, C07C 255/60

(54) **A new process for the preparation of irbesartan**

(30) Priority: 08.12.2008 SI 200800301
(71) Applicant: KRKA, tovarna zdravil, d.d., Novo mesto, 8501 Novo mesto (SI)
(72) Inventor: Zupancic, Silvo, 8000 Novo mesto (SI); Bevk, David, 8000 Novo mesto (SI); Zupet, Rok, 1211 Ljubljana (SI)
(74) Representative: HOFFMANN EITLE

(57) **Abstract**

A process for the preparation of Irbesartan or a pharmaceutically acceptable salt thereof comprising the step of coupling the 1-pentanamidocyclapentanecarboxamide of formula (V) with 4'-substituted methyl biphenyl-2-carbonitrile or 1-(4'-substituted methyl biphenyl-2-yl)-1H-tetrazole wherein tetrazole can be protected or unprotected to obtain the N-[(2'-cyanobiphenyl-4-yl)methyl]-1-pentanamidocyclopentanecarboxamide or *N*-[1-({[2'-(1*H-*tetrazol-1-yl)biphenyl-4-yl]methylamino)methyl)cydopentyl]pentanamide wherein tetrazole can be protected or unprotected that is further processed to Irbesartan or a pharmaceutical acceptable salt thereof.

## Description

### Field of the invention

The present invention describes a novel process for the preparation of Irbesartan and its pharmaceutically acceptable salts.

### Background of the Invention

lrbesartan is a non-peptide tetrazole derivative having an angiotensin II type 1 receptor (AT₁) antagonistic activity and is used in the treatment of hypertension.

Its chemical name is 2-Butyl-3-{[2'-(1*H*-tetrazol-5-yl)biphenyl-4-yl]methyl}-1,3-diazaspiro[4.4]non-1-en-4-one and it is represented by the following formula (I):

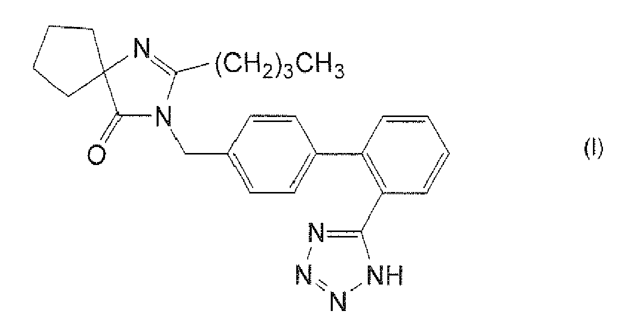

The empirical formula is C₂₅H₂₈N₆O and its molecular weight is 428.53 g/mol.

Processes for the preparation of lrbesartan are described in e.g. EP 0 454 511, which is the basic product patent, EP 0 475 898, EP 1 590 343, WO 2005/113518, WO 2007/122508 and WO 20071052301.

In EP 0 454 511 there are two principal preparation processes described. In the first process the nitrogen-containing spiro substituent is prepared in advance and then reacted with a 4-halomethylbiphenyl moiety. In the second process, a 4-aminomethylbiphenyl moiety is reacted with an accordingly substituted amino acid and the resulting product is then cyclized using an alkyl ortho-ester.

EP 0 475 898 discloses a preparation process of lrbesartan wherein 1-carboxy-l-pentanoyiamino-cyclopentan is reacted with 4-aminomethyl-2'-cyanobiphenyl and the obtained 1-(2'-cyanobiphenyl-4-ylmethylaminocarbonyl)-l-pentanoylamino-cyclopentan is cyclicized and further processed to Irbesartan.

In EP 1 590 343 an lrbesartan precursor 2-butyl-3-{[2'-(1-trityl-1*H*-tetrazol-5-yl)biphenyl-4-yl]methyl}-1,3-diazaspiro[4.4]non-1-en-4-one is prepared by
a) reacting in an one-pot set-up 1-pentanaminocyclopentanecarboxamide with 5-(4'-bromomethylbiphenyl-2-yl)-1-trityl-1*H*-tetrazole in the presence of an inorganic base and a phase transfer catalyst;
b) reacting a valerimidate derivative, especially ethyl valerimidate or a salt thereof, with a first amine, especially 5'-(4'-aminomethylbiphenyl-2-yl)-1-trityl-1*H*-tetrazol or 1-aminocyclapentane carboxylic acid ethyl ester to form a mixture and combining the mixture with a second amine especially 5'-(4'aminomethylbiphenyl-2-yl)-1-trityl-1*H*-tetrazole or 1-aminacyclopentane carboxylic acid ethyl ester (with the proviso that first and second amines are not the same); or
c) combining a valeramide derivative, especially ethyl valerimidate, with a base scavenger, especially 2,6-lutidine, and oxalyl chloride in the presence of an organic solvent, whereby an indoyl chloride intermediate is presumed to form, and further combining an amine, especially 5'-(4'aminomethylbiphenyl-2-yl)-1-trityl-1*H*-tetrazole or 1-amino-cyclopentane carboxylic acid ethyl ester with the combination;
which irbesartan precursor can be converted to Irbesartan by removing the trityl group.

In WO 2005/113518 Irbesartan is prepared from 4'-[(2-butyl-4-oxo-1,3-diazaspiro[4.4]non-1-en-3-yl)methyl]biphenyl-2-carbonitrile by reaction with sodium azide. 4'-[(2-butyl-4-oxo-1,3-diazaspiro[4.4]non-1-en-3-yl)methyl]biphenyl-2-carbanitrile in turn is prepared by reacting 1-aminocyclopentane carboxylic acid with valeroyl chloride and reacting the obtained pentanamidocyclopentane carboxylic acid with 4'-(aminomethyl)biphenyl-2-carbonitrile followed from cyclization to form 4'-[(2-butyl-4-oxo-1,3-diazaspiro[4.4]non-1-en-3-yl)methyl]biphenyl-2-carbonitrile.

In a similar manner, WO 2007/122508 discloses the preparation of Irbesartan via 4'-[(2-butyl-4-oxo-1,3-diazaspiro[4.4]nan-1-en-3-yl)methyl]biphenyl-2-carbonitrile by condensing pentanoylaminocyclopentane carboxylic acid with 4'-aminomethyl-2-cyanobiphenyl and reacting the obtained ring-open analog of 4'-[(2-butyl-4-oxo-1,3-diazaspiro[4.4]non-1-en-3-yl)methyl]biphenyl-2-carbonitrile with trialkyltin chloride and an alkaline azide.

In WO 2007/052301 there is a preparation process of Irbesartan disclosed, which comprises reacting 2-(n-butyl)-3-oxa-1-azaspiro[4.4]non-1-en-4-one with 4-aminomethyl-2'-cyanobiphenyl followed from cyclization of the obtained (ring-open) 1-(2'-cyanobiphenyl-4-ylmethylaminocarbonyl)-1-pentanoylaminocyclopentane to give 2-(n-butyl)-3-(2'-cyanobiphenyl-4-ylmethyl)-4-oxo-1,3-diazaspiro[4.4]non-1-ene, which is converted to lrbesartan.

Regardless of these various art known preparation processes, there still exists a need for an efficient synthesis of Irbesartan, which provides for mild reaction conditions and which does not require further cumbersome purification steps, such as chromatography in particular.

### Summary of the invention

The present invention relates to a novel process for the preparation of compounds useful in the preparation of Irbesartan or a pharmaceutical acceptable salt thereof comprising the step of *N*-alkylation of primary amide group of 1-pentanamidocyclopentanecarboxamide of formula (V) with 4'-substituted methyl biphenyl-2-carbonitrile of formula (IVa), wherein X is a halogen atom or a leaving group, such as for example Cl, Br, I, tosylate, mesylate or triflate, to obtain N-[(2'-cyanabiphenyl-4-yl)methyl]-1-pentanamidocyclopentanecarboxamide of formula (IIIa). 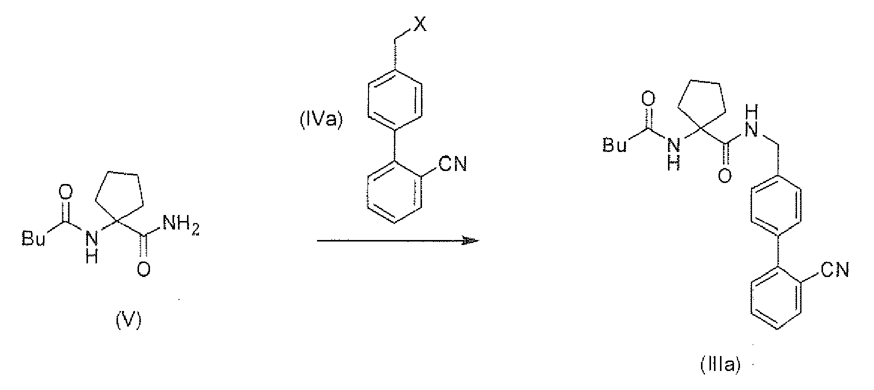 In another embodiment the present invention relates to a novel process for the preparation of compounds useful in the preparation of Irbesartan or a pharmaceutical acceptable salt thereof comprising the step of *N*-alkylation of primary amide group of 1-pentanamidocyclopentanecarboxamide of formula (V) with a 1-(4'-substituted methyl biphenyl-2-yl)-1 H-tetrazole wherein said tetrazole can be protected or unprotected of formula (IVb) and wherein X is a halogen atom or a leaving group, such as for example Cl, Br, I, tosylate, mesylate or triflafie, to obtain *N*-[1-({[2'-(1*H*-tetrazol-1-yl)biphenyl-4-yl]methylamino}methyl)cyclopentyl]pentanamide of formula (IIIb): 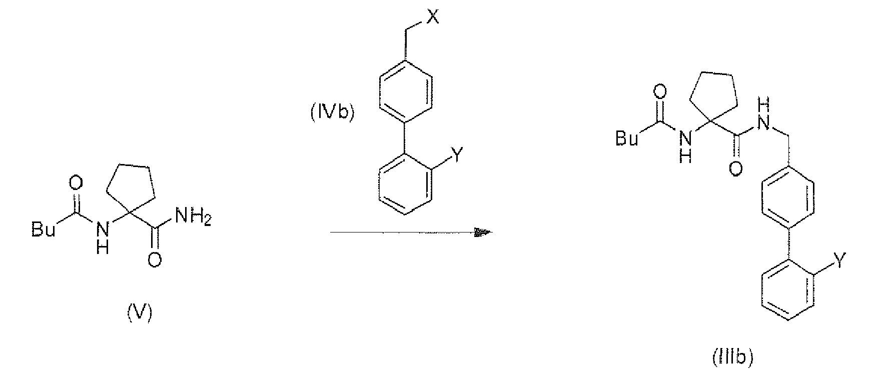 wherein Y means protected or unprotected tetrazole group.

In another embodiment the present invention relates to novel processes for the preparation of Irbesartan or a pharmaceutical acceptable salt thereof comprising the either one or the other of the above reaction steps. A preferred variant of this embodiment comprises the steps of:
a) *N*-alkylation of primary amide group of 1-pentanamidocyclopentanecarboxamide of formula (V) with 4'-substituted methyl biphenyl-2-carbonitrile of formula (IVa), wherein X is halogen atom or leaving group such as for example Cl, Br, I, tosylate, mesylate or triflate to obtain *N*-[(2'-cyanobiphenyl-4-yl)methyl]-1-pentanamidocyclopentanecarboxamide of formula (IIIa) 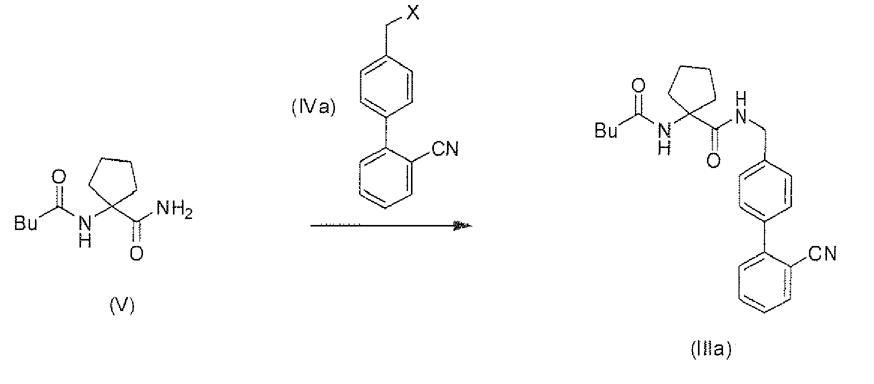
b) cyclization of the pentanamidocyclopentanecarboxamide moiety of *N*-[(2'-cyanobiphenyl-4-yl)methyl]-1-pentanamidocyclopentartecarboxamide of formula (IIIa) to obtain 4'-[(2-butyt-4-oxo-1,3-diazaspiro[4.4]non-1-en-3-yl)methyl]biphenyl-2-carbonitrile of formula (IIa): 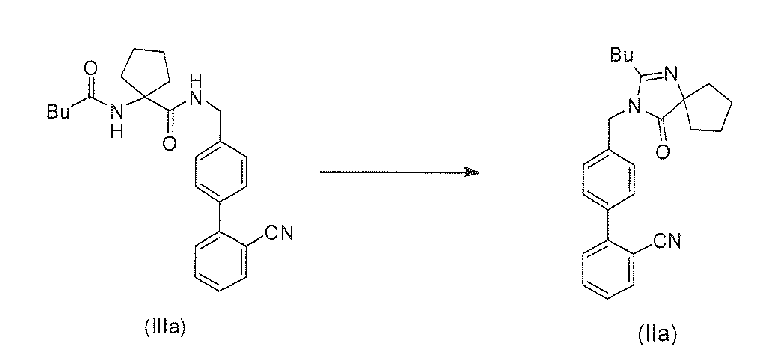
c) converting the carbonitrile moiety of 4'-[(2-butyl-4-oxo-1,3-diazaspiro[4.4]non-1-en-3-yl)methyl]biphenyl-2-carbonitrile of formula (IIa) with sodium azide into a tetrazolyl moiety to provide Irbesartan (I): 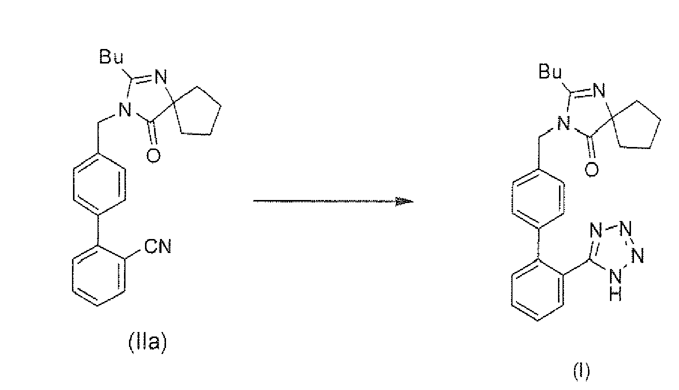
d) optionally converting Irbesartan into one of its pharmaceutically acceptable salts, preferably Irbesartan-HCl (Ia):

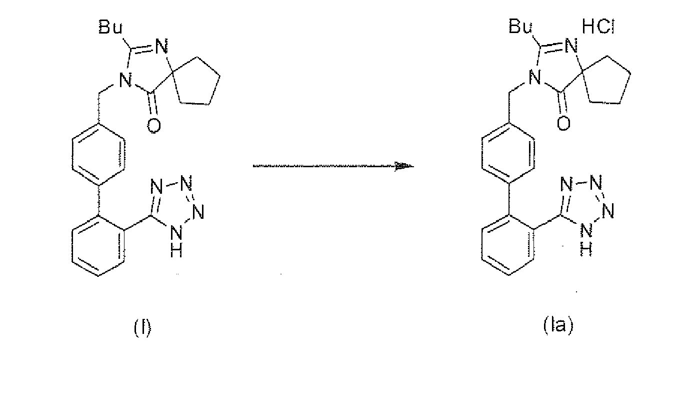

In another embodiment the present invention relates to a process for the preparation of Irbesartan or a pharmaceutical acceptable salt thereof as specified in step (c) above, wherein said conversion is conducted with sodium azide in a basic organic solvent and said conversion is catalyzed by addition of an acid.

In another embodiment the present invention relates to a process for the preparation of a pharmaceutical composition comprising the steps of preparing Irbesartan or a pharmaceutically acceptable salt thereof by a process according to any of the preceding embodiments and mixing a therapeutically effective amount thereof optionally with one or more other active substances and with one or more pharmaceutically acceptable excipients.

### Detailed description of the invention

The present inventors surprisingly found that a 4'-substituted methyl biphenyl-2-carbonitrile compound or a 1-(4'-substituted methyl biphenyl-2-yl)-1*H*-tetrazole compound, wherein tetrazole is protected or unprotected, selectively reacts with the primary carboxamide moiety of 1-pentanamidocyclopentanecarboxamide by way of a nucleophilic substitution in the presence of a base. Interestingly, the reaction proceeds very well under very mild conditions, it does not require cumbersome purification steps such as column chromatography and it gives the desired intermediate with high purity. Simple recrystallization from an appropriate solvent or solvent mixture, preferably acetone, already provides the desired intermediate in a good yield. Moreover, in contrast to some prior art methods, the use of a phase transfer catalyst is not required and, hence, in a preferred embodiment, the coupling step takes place in the absence of such a phase transfer catalyst.

In addition, the reaction starts from ready available and inexpensive starting compound that is widely used in the synthesis of different sartans, e.g. candesartan, telmisartan, losartan, olmesartan and valsartan. Moreover, in contrast to prior art methods the base and the solvent(s) used in the process are easy to handle and unharmful to health and environment.

Accordingly, the present invention relates to a novel processes for the preparation of Irbesartan or a pharmaceutical acceptable salt thereof and reaction intermediates suitable for manufacturing the same.

According to the present invention, Irbesartan can be in the form of the free substance or a pharmaceutically acceptable salt thereof. Suitable pharmaceutically acceptable salts are hydrochlorides, sulphates, phosphates and hydrobromides. Irbesartan and/or its pharmaceutically acceptable salts as obtained according to the methods of the present invention can be in the form of the amorphous substance or any crystalline (polymorphic) form thereof. Moreover, it is contemplated in the context of the present invention to prepare any pharmaceutically acceptable solvate (hydrate) form of Irbesartan and its pharmaceutically acceptable salts.

According to one embodiment of the invention the inventive processes comprise the step of *N*-alkylation of primary amide group of 1-pentanamidocyclopentanecarboxamide of formula (V) with 4'-substituted methyl biphenyl-2-carbonitrile of formula (IVa), wherein X is a halogen atom or a leaving group, to obtain N-[(2'-cyanobiphenyl-4-yl)methyl]-1-pentanamidocyclopentanecarboxamide of formula (IIIa):

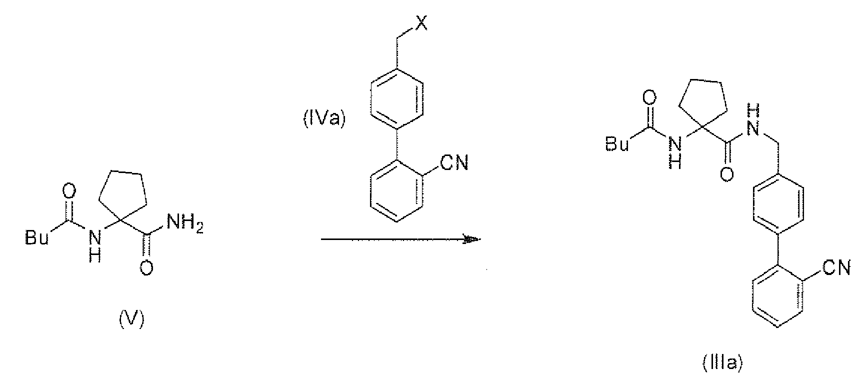

The term "leaving group", as it is used in the present invention, refers to a functional group characterized by an ability to detach itself from a molecule Preferred leaving groups are OTs (tosylate), OMs (mesylate) and OTf (trifilate). Preferred halogen atoms are Cl, Br, and I.

The obtained N-[(2'-cyanobiphenyl-4-yl)methyl]-1-pentanamidocyclopentanecarboxamide of formula (IIIa) is well suited to be further processed to Irbesartan or a pharmaceutical acceptable salt thereof.

Another embodiment of the present invention relates to methods for the preparation of irbesartan or pharmaceutically acceptable salts thereof, comprising the above process as a reaction step.

In a further preferred embodiment of the present invention the 4'-substituted methyl biphenyl-2-carbonitrile of formula (IVa) is 4'-(halomethyl)biphenyl-2-carbonitrile and more preferably 4'-(bromomethyl)biphenyl-2-carbonitrile.

In a further preferred embodiment of the present invention N-[(2'-cyanobiphenyl-4-yl)methyl]-1-pentanamidocyclopentanecarboxamide of formula (IIIa) has a purity of at least 97%, preferably of at least 98% and more preferably of at least 99%.

In another embodiment the present invention relates to a novel process for the preparation of Irbesartan or a pharmaceutical acceptable salt thereof comprising the step of *N*-alkylation of primary amide group of 1-pentanarnidocyclopentanecarboxarnide of formula (V) with 1-(4'-substituted methyl biphenyl-2-yl)-1H-tetrazole wherein tetrazole can be protected or unprotected of formula (IVb) and wherein X is a halogen atom or a leaving group such as for example Cl, Br, I or OTs (tosylate) or OMs (mesylate) or OTf (triflate) to obtain *N*-[1-({[2'-(1*H-*tetrazol-1-yl)biphenyl-4-yl]methylamino}methyl)cyclopentyl]pentanamide of formula (IIIb): 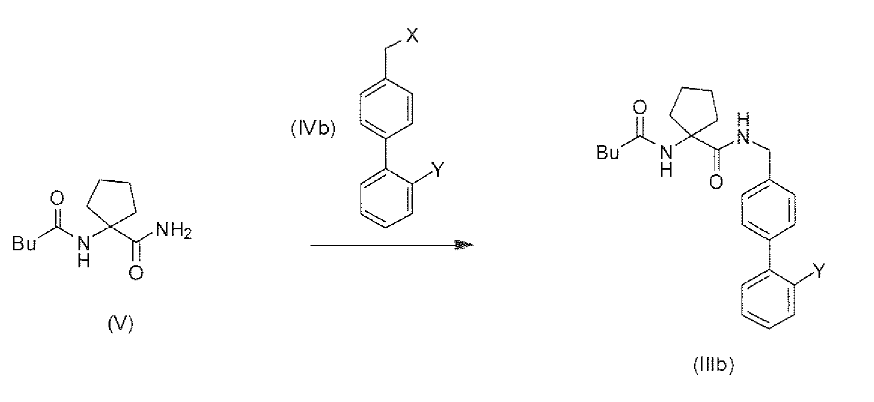 wherein Y means protected or unprotected tetrazole group. The leaving group X is as defined above.

Suitable protecting group for the tetrazole group is trityl group.

The obtained compound of formula (IIIb) is well suited to be further processed to Irbesartan or a pharmaceutical acceptable salt thereof.

Another embodiment of the present invention relates to methods for the preparation of Irbesartan or pharmaceutically acceptable salts thereof, comprising the above process as a reaction step.

If group Y in compound (IIIb) in the above reaction is a protected tetrazole group, the synthesis of irbesartan also includes a subsequent step of deprotection. This deprotection step is preferably carried out before or after the step of cyclization yielding the 4-oxo-1,3-diazaspiro[4,4]non-1-en-3-yl group. Suitable deprotection agents and reaction conditions are as follows: mineral acids, organic acids, and strong bases.

In a further preferred embodiment of the present invention the 1-(4'-substituted methyl biphenyl-2-yl)-1*H*-tetrazole of formula (IVb) is N-protected 5-(4'-(bromomethyl)biphenyl-2-yl)-1*H*-tetrazole and more preferably 5-(4'-(bromomethyl)biphenyl-2-yl)-1*H*-tetrazole.

In a further preferred embodiment of the present invention the compound of formula (IIIb) is *N*-{[2'-(1*H*-tetrazol-5-yl)biphenyl-4-yl]methyl}-1-pentanamidocyclopentane-carboxamide and 1-pentanamido-*N*-{[2'-(1-trityl-1*H*-tetrazol-5-yl)biphenyl-4-yl]methyl}cyclopentanecarboxamide.

In a further preferred embodiment of the present invention the *N*-[1-({[2'-(1*H*-tetrazol-1-yl)biphenyl-4-yl]methylamino}methyl)cyclopentyl]pentanamide of formula (IIIb), wherein the tetrazole group can be protected or unprotected, is purified to a degree of at least 97%, preferably of at least 98% and more preferably of at least 99%.

In a preferred embodiment of the present invention *N*-alkylation of 1-pentanamidocyclopentanecarboxamide of formula (V) with the compound of formula (IVa) or (IVb) is performed in the presence of a base that can be selected from, but not limited to, metal hydroxides such as for example NaOH, KOH, Ca(OH)₂, preferably KOH.

In a preferred embodiment of the present invention *N*-alkylation of 1-pentanamidocyclopentanecarboxamide of formula (V) with the compound of formula (IVa) or (IVb) is performed in the presence of one or more iodide salts that can be selected from, but not limited to, Nal, KI, Lil, NH₄I, preferably Kl.

In a preferred embodiment of the present invention *N*-alkylation of the primary amide group of 1-pentanamidocyclopentanecarboxamide of formula (V) with the compound of formula (IVa) or (IVb) is conducted in an organic solvent or any solvent mixture containing one or more organic solvents, such as for example acetonitrile, at a temperature between 15 and 30°C, preferably at room temperature.

In another preferred embodiment of the processes of the present invention, purification of the compound of formula (IIIa) or (IIIb) obtained by *N*-alkylation of primary amide group of 1-pentanamidocyclopentanecarboxamide of formula (V) with the compound of formula (IVa) or (IVb) is conducted by recrystallization.

In a further preferred embodiment of the present invention said recrystallization is conducted in an organic solvent or any solvent mixture containing one or more organic solvent, such as for example acetone. The yield of the reaction and recrystallization is at least 65%, preferably at least 70 % and more preferably at least 80 % is achieved calculated according to compound (IVa) or (IVb).

The further conversion of the compound of formula (IIIa) to Irbesartan or a pharmaceutical acceptable salt thereof can be carried out by methods known *per se*; the most preferable method is intramolecular cyclization of the pentanamidocyclopentanecarboxamidyl moiety and conversion of the nitrile group into a tetrazolyl group, wherein the order of these two reaction steps preferably is (1) intramolecular cyclization and (2) conversion of the nitrile group into a tetrazolyl group using a suitable azide reagent, preferably sodium azide. However, in another embodiment of the invention this sequence may also be inverted, i.e. (1) preparation of the tetrazolyl group and (2) intramolecular cyclization.

The further conversion of the compound of formula (IIIb) to Irbesartan or a pharmaceutical acceptable salt thereof can be carried out by methods known *per se*; such as for example disclosed in J. Med. Chem., 1993, 36, 3371-3380 or WO2006/073376, the most preferable method is intramolecular cyclization of the pentanamidocyclopentanecarboxamidyl moiety and alternatively removing tetrazol protecting group.

Preferred reagents and reaction conditions for the above-mentioned cyclization reactions are described below.

In another embodiment the present invention relates to a novel process for the preparation of Irbesartan or a pharmaceutical acceptable salt thereof comprising the steps of:
a) *N*-alkylation of primary amide group of 1-pentanamidocyclopentanecarboxamide of formula (V) with 4'-substituted methyl biphenyl-2-carbonitrile of formula (IVa), wherein X is halogen atom or leaving group such as for example Cl, Br, I, OTs (tosylate), OMs (mesylate) or OTf (triflate) to obtain *N*-[(2'-cyanobiphenyl-4-yl)methyl]-1-pentanamidocyclopentanecarboxamide of formula (IIIa) 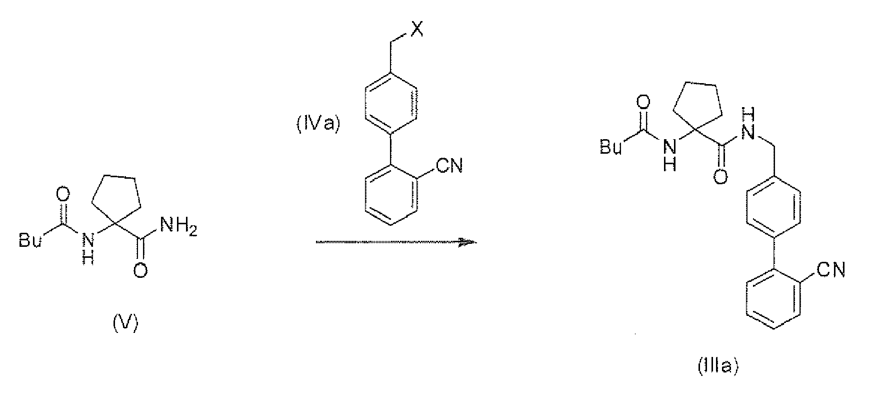
b) cyclization of the pentanamidocyclopentanecarboxamide moiety of N-[(2'-cyanobiphenyl-4-yl)methyl]-1-pentanamidocyclopentanecarboxamide of formula (IIIa) to obtain 4'-[(2-butyl-4-oxo-1,3-diazaspiro[4.4]non-1-en-3-yl)methyl]biphenyl-2-carbonitrile of formula (IIa): 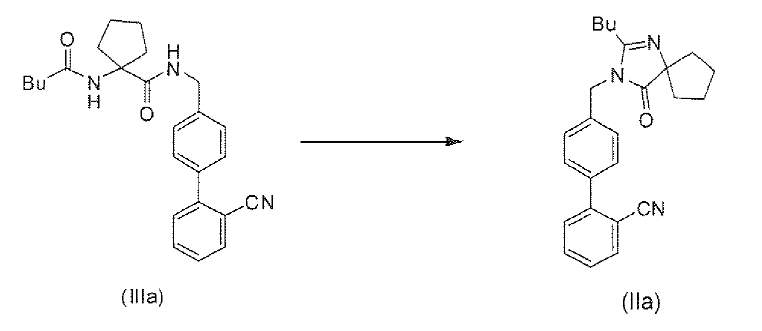
c) converting the carbonitrile moiety of 4'-[(2-butyl-4-oxo-1,3-diazaspiro[4.4]non-1-en-3-yl)methyl]biphenyl-2-carbonitrile of formula (IIa), preferably with sodium azide, into a tetrazolyl moiety to provide Irbesartan (I): 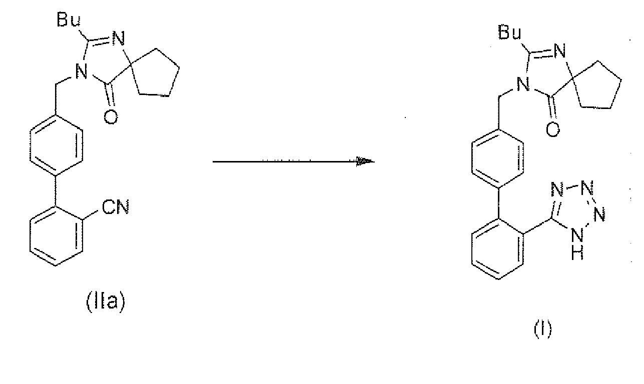
d) optionally converting Irbesartan into one of its pharmaceutically acceptable salts, preferably Irbesartan-HCl (1a):

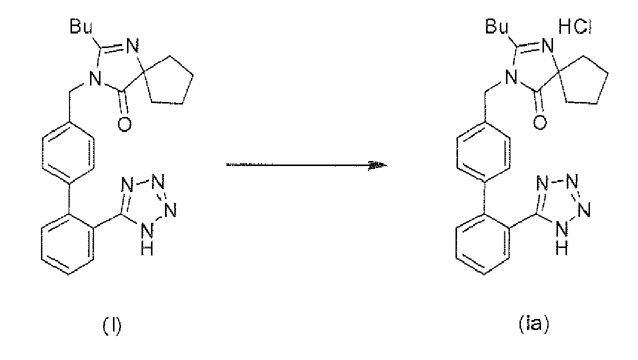

In a preferred embodiment of the present invention the *N*-alkylation of primary amide group of 1-pentanamidocyclopentanecarboxamide of formula (V) with 4'-substituted methyl biphenyl-2-carbonitrile of formula (IVa) is performed according to the previous embodiments of the present invention.

In a preferred embodiment of the present invention the starting compound 1-pentanamidocyclopentanecarboxamide of formula (V) can be prepared by acylation of amino group of 1-aminocyclopentanecarboxamide of formula (VI) with valeroyl chloride to obtain 1-pentanamidocyclopentanecarboxamide of formula (V).

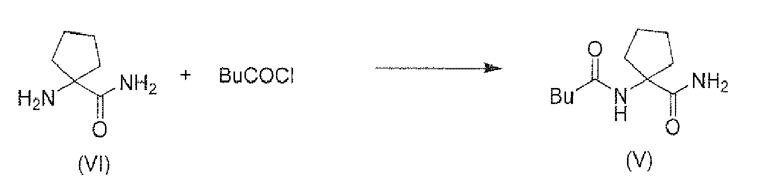

In a further preferred embodiment of the present invention the acylation of amino group of 1-aminocyclopentanecarboxamide of formula (VI) with valeroyl chloride to obtain 1-pentanamidocyclopentanecarboxamide of formula (V) is performed in an organic solvent, preferably in dichloromethane at a temperature between -10°C and 20°C and preferably at 0°C to 10°C.

Alternatively, the starting compound 1-pentanamidocyclopentanecarboxamide of formula (V) can be prepared by acylation of the amino group of 1-amino-1-cyanocyclopentane of formula (VIII) with valeroyl chloride to give *N*-(1-cyanocyciopentyl)pentanamide of formula (VII), followed by hydrolysis of the cyano group to furnish 1-pentanamidocyclopentanecarboxamide of formula (V).

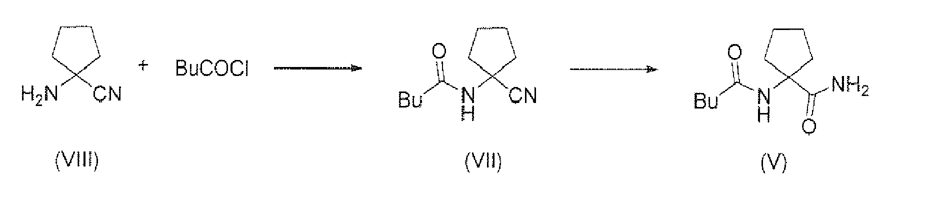

Afternatively, the starting compound 1-pentanamidacyclopentanecarboxamide of formula (V) can be prepared according to any process known from the prior art such as for example disclosed in US 5763661, WO 2006089927, CN 1429817, WO 2004072064, CA 2154807, WO 9114679.

In a preferred embodiment of the present invention cyclization of the pentanamidocyclopentanecarboxamide moiety of N-[(2'-cyanobiphenyl-4-yl)methyl]-1-pentanamidocyclopentanecarboxamide of formula (IIIa) is performed in an inert solvent such as for example aromatic or non-aromatic carbohydrates such as for example toluene, xylenes and optionally in the presence of basic compounds such as for example pyridine or triethylamine or in the presence of acidic compounds such as for example mineral acid or organic carbo- or sulfone-acid such as for example hydrochloric acid, hydrobromic acid, acetic acid, *p*-toluene, sulfonic acid, methane sulfonic acid 2-propanephosphonic acid anhydride (T3P), preferably in trifluoroacetic acid.

In a further preferred embodiment of the present invention cyclization of the pentanamidocyclopentanecarboxamide moiety of N-[(2'-cyanabiphenyl-4-yl)methyl]-1-pentanamidocyclopentanecarboxamide of formula (IIIa) is conducted in an organic solvent or any mixtures thereof preferably in toluene at a temperature between 100 and 115°C and preferably at reflux temperature.

The same reaction conditions may also be employed for the cyclization of the compound of formula (IIIb).

In a preferred embodiment of the present invention the conversion of 4'-[(2-butyl-4-oxo-1,3-diazaspiro[4.4]non-1-en-3-yl)methyl]biphenyl-2-carbonitrile of formula (IIa) to obtain Irbesartan (I) is performed in an basic organic solvent, preferably in 1-methylimidazole. Suitable basic solvents should have a pK_{B} of less than 7, preferably between 7.0 and 4.0 and a boiling point of at least 120°C. Solvent mixtures can also be employed. 1-Methylimidazole is preferred because it can be easily recycled, it has high boiling point and the reaction provides high yield. The same solvent can also be used when forming the tetrazole group prior to cyclization.

In a further preferred embodiment of the present invention the conversion of 4'-[(2-butyl-4-oxo-1,3-diazaspiro[4.4]non-1-en-3-yl)methyl]biphenyl-2-carbonitrile of formula (IIa) to obtain Irbesartan (I) is conducted with sodium azide that is environmentally friendly because of its easy neutralization. The same sodium azide can also be used when forming the tetrazole group prior to cyclization.

In a further preferred embodiment of the present invention the conversion of 4'-[(2-butyl-4-oxo-1,3-diazaspiro[4.4]non-1-en-3-yl)methyl]biphenyl-2-carbonitrile of formula (IIa) to obtain Irbesartan (I) is catalyzed by addition of an acid such as organic or inorganic mineral acid, Lewis or Bronsted acid. Preferably acetic acid, hydrochloric acid or triethylamine hydrochloride can be added. The same catalysts can also be used when forming the tetrazole group prior to cyclization.

In a further preferred embodiment of the present invention the conversion of 4'-[(2-butyl-4-oxo-1,3-diazaspiro[4.4]non-1-en-3-yl)methyl]biphenyl-2-carbonitrile of formula (IIa) to obtain Irbesartan (I) is conducted at a temperature between 80°C and 170°C and preferably between 100 to 140°C. Such reaction temperatures are particularly favourable when using a basic organic solvent such as 1-methylimidazole. The same reaction temperatures can also be used when forming the tetrazole group prior to cyclization.

In a preferred embodiment of the present invention Irbesartan is converted into one of its pharmaceutically acceptable salts, preferably Irbesartan-HCl (Ia). This conversion can be conducted according to any known processes such as for example described in WO 2006011859.

In another embodiment the present invention relates to Irbesartan or a pharmaceutical acceptable salt thereof prepared according to one of the above processes for use in a method for treating hypertension.

In another embodiment the present invention relates to a process for the preparation of a pharmaceutical composition comprising the steps of preparing Irbesartan or a pharmaceutical acceptable salt thereof according to one of the above processes and mixing a therapeutically effective amount thereof with one or more pharmaceutically acceptable excipients and optionally with one or more further active substances. Preferred examples of such further active substances are selected from the group of antihypertensives that can be selected from the group of angiotensive converting enzyme (ACE) inhibitors, angiotensin receptor blockers (ARBs), calcium channel blockers (CCBs) and antiadrenergics. Lipid regulators can for example be HMG CoA reductase inhibitors, diuretics such as hydrochlorothiazide (HCTZ) or furosemide, lipid regulators or antidiabetics. According to the present invention, HCTZ preferably has an average particle size of less than 80 µm, preferably less than 50 µm, more preferably less than 30 µm.

The term "average particle size" as used herein refers to the volume mean diameter of the particles. The volume mean diameter was determined by laser light scattering using a Malvern-Mastersizer Apparatus MS 2000 equiped with Hydro S dispersion unit. Vegetable oil was used as the dilution medium.

A therapeutically effective amount of irbesartan or a pharmaceutically acceptable salt thereof are preferably provided in a unit dosage form, each unit dosage form containing from about 1-500 mg, preferably 1-300 mg, and even more preferably 75-300 mg of irbesartan in its free form (e.g. therapeutic dose of 300 mf free irbesartan corresponds to 344.43 mg of irbesartan-HCl*1.5 H₂O).

It has surprisingly been found that the stability of the irbesartan composition according to the invention is not adversely affected if the other active ingredient is selected from the group consisting of captopril, enalapril, cilazapril, lisinopril, trandolapril, ramipril, fosinopril, perindopril, amlodipine, diltiazem, felodipine, nifedipine, nitrendipine, verapamil, acebutol, atenolol, betaxolol, bisoprolol, metoprolol, carvedilol, lovastatin, simvastatin, pravastatin, atorvastatin, fluvastatin, rosuvastatin, indapamide, hydrochlorothiazide, sulfonyl urea, metglinides such as nateglinide or repaglinide, thiazolidinediones such as pioglitazone or rosiglitazone, α-glucosidase inhibitors, incretin mimetics, biguanides such as metformin hydrochloride and pharmaceutically acceptable salts thereof.

The pharmaceutically acceptable excipients used in and for the preparation of the present pharmaceutical composition particularly include diluents, binders, disintegrants and lubricants. Other and further excipients can also be contained.

The pharmaceutical composition can be used for oral administration such as in the form of tablets, coated tablets, capsules, sachet and oral liquids, or can be used for topical administration such as in the form of gels, lotions, patches, or ointments, or can be used for parenteral administration such as intravenous, intramuscular, or subcutaneous injection, or can be used as suppositories, .

In a preferred embodiment the pharmaceutical composition is for the treatment of hypertension.

The present invention is described in more detail by the following examples, but is not limited thereto.

### Example 1

### 1-Pentanamidocyclopentanecarboxamide

### a)

11.5 g (90 mmol) of 1-aminocyclopentanecarboxamide were suspended in 16 ml of methylene chloride. Then, 270 ml of a 25% solution of K₂CO₃ were added and the mixture was cooled to 0-5°C. At this temperature, 13 ml (108 mmol) of valeroyl chloride were slowly added while vigorously stirring. The reaction mixture was stirred for 2h at 0-5°C and 1h at room temperature Then, the product was filtered and washed with water and methanol. The wet product was dried. Yield: 18g (99%).

### b)

1-Amino-1-cyanocyclopentane (1.10 g) is dissolved in toluene (11 ml), triethytamine (1.5 mi) was added and valeroyl chloride (1.2 ml) was added to this solution dropwise, under cooling at 20°C. After 10-15 min the temperature was raised to 80°C and the mixture was stirred at that temperature for about 2 h. The reaction mixture was then cooled to room temperature and was extracted consecutively with water (5 ml), 2% hydrochloric acid (5 ml) and water (5 ml). The toluene solution was evaporated under vacuo to give *N*-(1-cyanocyclopentyl)pentanamide (1.94 g, 100%) as white powder.
Reference: Bioorg. Med. Chem., 2007, 15, 7391.

*N*-(1-cyanocyclopentyl)pentanamide (0.194 g) was dissolved in ethanol (5 ml) and sodium hydroxide (5 ml, 1M). While stirring, hydrogen peroxide (10 ml, 30%) was added dropwise and stirred at room temperature for 20 h. Ethanol was evaporated, water was added (15 ml) and extracted with dichloromethane (3×10 ml). Combined organic phases were dried and evaporated to dryness to give 1-pentanamidocyclopentanecarboxamide (0.20 g, 94%) as white powder.

### Example 2

### N-[(2'-Cyanobiphenyl-4-yl)methyl]-1-pentanamidocyclopentane-carboxamide

### a)

2.55 g (12 mmol) of 1-pentanamidocyclopentanecarboxamide were dissolved in 40 ml of acetonitrile at room temperature. Then, 2.72 g (10 mmol) of 4'-(bromomethyl)biphenyl-2-carbonitrile and 2.2 g of powdered KOH were added and the mixture was stirred for 2h at room temperature. The reaction mixture was diluted with 140 ml of water and neutralized by adding 1M HCl (25 ml, pH = 4.41). The precipitated product was filtered and washed with fresh water. The isolated crude product was dried. Yield: 3.64g.
The crude product was recrystallized from acetone.
Yield: 2.8g (69%) HPLC Area %: 99.22%

### b)

7.40 g (35 mmol) of 1-pentanamidocyclopentanecarboxamide were dissolved in 115 ml of acetonitrile at room temperature. Then, 7.89 g (29 mmol) of 4'-(bromomethyl)biphenyl-2-carbonitrile and 6.4 g of powdered KOH were added and the mixture was stirred for 2h at room temperature. The reaction mixture was diluted with 300 ml of water and neutralized by adding 1M HCl (72 ml, pH = 4.66). The precipitated product was filtered and washed with fresh water. The isolated crude product was dried. Yield: 10.7g.
The crude product was recrystallized from acetone.
Yield: 8.94 (76%) HPLC Area %: 97.61%

### c)

16.9 g (80 mmol) of 1-pentanamidocyclopentanecarboxamide were dissolved in 200 ml of acetonitrile at room temperature. Then, 18.0 g (64.3 mmol) of 4'-(bromomethyl)biphenyl-2-carbonitrile, 14,6 g of powdered KOH, 12.1 g of potassium iodide were added and the mixture was stirred for 2h at room temperature. The reaction mixture was diluted with 500 ml of water and neutralized by adding 1 M HCl (58 ml) to pH 4-5. The precipitated product was filtered and washed with fresh water. The isolated crude product was dried. Yield: 25 g.
The crude product was recrystallized from acetone.
Yield: 21.2g (82 %) HPLC Area %: 98.60

### Example 3

### 4'-[(2-Butyl-4-oxo-1,3-diazaspiro[4.4]non-1-en-3-yl)methyl]biphenyl-2-carbonitrile

### a)

A mixture of 5.6g (13.9 mmol) of N-[(2'-cyanobiphenyl-4-yl)methyl]-1-pentanamidocyclopentanecarboxamide, 80 ml of toluene and 2.4 ml of trifluoroacetic acid was heated to reflux temperature. The mixture is stirred at this temperature for 18h. During this process water is separated from the mixture by distillation. The mixture was cooled and 10 ml of water were added. The pH value of the mixture was adjusted to 6.40 by adding 1 M NaOH. Phases were separated and the organic phase was washed twice with water (20 ml). The organic phase was concentrated to an oily residue. This residue was suspended in *tert-*butyl methyl ether and cooled to 0°C. The precipitated product was filtered and washed with *tert*-butyl methyl ether and dried.
Yield: 4.55 g (85 %) HPLC Area %: 96.71%

### b)

A mixture of 20.2 g (50 mmol) of N-[(2'-cyanobiphenyl-4-yl)methyl]-1-pentanamidocyclopentanecarboxamide, 290 ml of toluene, 8.6 ml (112 mmol) of trifluoroacetic acid was heated to reflux temperature. The mixture is stirred at this temperature for 24h. During this process water is separated from the mixture by distillation. The mixture was cooled and 82 ml of 1M NaOH were added to adjust pH to a value between 8 and 11. Phases were separated and the organic phase was washed twice with water (300 ml). The organic phase was concentrated to an oily residue. This residue was suspended in *tert*-butyl methyl ether (12 ml) and cooled to 0°C. The precipitated product was filtered and washed with *tert*-butyl methyl ether and dried.
Yield: 17.4g (90 %) HPLC Area %: 95.98.

### c)

A mixture of 4.43 g (11 mmol) of N-[(2'-cyanobiphenyl-4-yl)methyl]-l-pentanamidocyclopentanecarboxamide, 64 ml of toluene and 2.33 g (12.3 mmol) of p-taluenesulfcanic acid monohydrate was heated to reflux temperature. The mixture is stirred at this temperature for 18h. During this process water is separated from the mixture by distillation. The mixture was cooled and 8 ml of water were added. The pH value of the mixture was adjusted to 6.59 by adding 1M NaOH. Phases were separated and the organic phase was washed twice with water (20 ml). The organic phase was concentrated to an oily residue. This residue was suspended in *tert*-butyl methyl ether and cooled to 0°C. The precipitated product was filtered and washed with *tert*-butyl methyl ether and dried.
Yield: 4.82g (90%) HPLC Area %: 98.43%

### d)

A mixture of 6.06 g (15 mmol of N-[(2'-cyanobiphenyl-4-yl)methyl]-1-pentanamidocyclopentanecarboxamide, 50 ml of toluene, 5 ml 50 % T3P in ethyl acetate was heated to reflux temperature. The mixture is stirred at this temperature for 30h. During this process water is separated from the mixture by distillation. The mixture was cooled and 57 ml of 1M NaOH were added to adjusted pH to between 8 and 11. Phases were separated and the organic phase was washed twice with water (90 ml). The organic phase was concentrated to an oily residue. This residue was suspended in *tert*-butyl methyl ether (6ml) and cooled to 0°C. The precipitated product was filtered and washed with *tert*-butyl methyl ether and dried.
Yield: 5.25 g (90 %) HPLC Area %: 97.09.

### Example 4

### Irbesartan hydrochloride

### Process A

4'-[(2-Butyl-4-oxo-1,3-diazaspiro[4.4]non-1-en-3-yl)methyl]biphenyl-2-carbonitrile (2.5 mmol, 0.96g) was dissolved in 1-methylimidazole (5 ml); sodium azide (5 mmol, 0.325g) and triethylamine hydrochloride (5 mmol, 0.685 g) were added and the reaction mixture was heated for 48 h at 130°C. The reaction mixture was then cooled to room temperature diluted with water (10 ml), acidified with hydrochloric acid, and extracted with dichloromethane (3×5ml). The combined organic phases were washed with water, the pH value was set to 10-12, and it was washed with dichloromethane (10 ml). The solution was acidified with hydrochloric acid to pH<1 and Irbesartan hydrochloride precipitated from the mixture.
Yield: 1.05 g (85%) HPLC Area %:99.0%

### Process B

4'-[(2-Butyl-4-oxo-1,3-diazaspiro[4.4]non-1-en-3-yl)methyl]biphenyl-2-carbonitrile (2.5 mmol, 0.96g) was dissolved in 1-methylimidazole (5 ml); sodium azide (5 mmol, 0.325g) and concentrated hydrochloric acid (0.5 ml) were added and the reaction mixture was heated for 24h at 160°C. After cooling the reaction mixture to room temperature, water was added (10 ml) and pH was set to 12-13 with sodium hydroxide (1 M). Mixture was extracted with ethyl acetate (2×5 ml). Water phase was then added dropwise to hydrochloric acid solution (20 ml, 1M) and product precipitated from mixture, which was filtered off and dried to give irbesartan hydrochloride.
Yield: 0.68 g (55%), HPLC Area %: 99,05%

### Process C

*N*-[(2-cyanobiphenyl-4-yl)methyl]-1-pentanamidocyclopentanecarboxamide (2.00 g, 5.2 mmol), triethylamine hydrochloride (1.426 g, 10.4 mmol) and sodium azide (0.674 g, 10.4 mmol) were dissolved in 1-methylpyrrolidin-2-one (2 ml) and heated 12h at 121-123°C. Reaction mixture was then cooled to 40-50°C, sodium hydroxide (10 ml, 35%) was added and stirred for 30 min at 20-40°C. Phases were separated, organic phase was treated with mixture water / toluene (5 ml / 2 ml) and mixture was stirred for additional 30 min at 20-30°C. Phases were separated, water phase was washed with ethyl acetate (5 ml). Solution of irbesartan sodium salt in water was added dropwise under vigorous stirring into hydrochloric acid solution at pH <1. Product precipitates from reaction mixture and was filtered off to give irbesartan hydrochloride.
Yield = 2.11g (83%), HPLC Area %:98.89%

### Process D

*N*-[(2'-cyanobiphenyl-4-yl)methyl]-1-pentanamidocyclopentanecarboxamide (0,964 g, 2.5 mmol), was dissolved in 1-methylimidazole, sodium azide (0.325 g, 5 mmol) and triethylamine hydrochloride (0.685 g, 5 mmol) were added to the solution and stirred 48 h at 130°C. After cooling the reaction mixture to room temperature, water was added (10 ml) and pH was set to 12-13 with sodium hydroxide (1 M). Mixture was extracted with ethyl acetate (2×5 ml). Water phase was then added dropwise to hydrochloric acid solution (20 ml, 1M) and product precipitated from mixture, which was filtered off and dried to give irbesartan hydrochloride.
Yield: 0.813 g (66%), HPLC Area %:99.10%

### Process E

*N*-[(2'-cyanobiphenyl-4-yl)methyl]-1-pentanamidocydopentanecarboxamide (2.5 mmol, 0.964 g) was dissolved in 1-methylimidazoie, sodium azide (5 mmol, 0.325 g) and hydrochloric acid solution in ethanol (5 mmol) were added and heated 24h at 160°C. After cooling the reaction mixture to room temperature, water was added (10 ml) and pH was set to 12-13 with sodium hydroxide (1 M). Mixture was extracted with ethyl acetate (2x5 ml). Water phase was then added dropwise to hydrochloric acid solution (20 ml, 1M) and product precipitated from mixture, which was filtered off and dried to give irbesartan hydrochloride.
Yield: 1.08 g (88%) HPLC Area %: 98.90 %

### Process F

*N*-[(2'-cyanobiphenyl-4-yl)methyl]-1-pentanamidocyclopentanecarboxamide (2.5 mmol, 0.964 g) was dissolved in 1-methylimidazole, sodium azide (5 mmol, 0.325 g) and acetic acid (0.5 ml) were added and heated 24h at 160°C. After cooling the reaction mixture to room temperature, water was added (10 ml) and pH was set to 12-13 with sodium hydroxide (1 M). Mixture was extracted with ethyl acetate (2×5 mi). Water phase was then added dropwise to hydrochloric acid solution (20 ml, 1M) and product precipitated from mixture, which was filtered off and dried to give irbesartan hydrochloride.
Yield: 1.10 g (90%) HPLC Area %: 99.10%

### Process G

4'-[(2-Butyl-4-oxo-1,3-diazaspiro[4.4]non-1-en-3-yl)methyl]biphenyl-2-carbonitrile (17.4 g; 45 mmol) was dissolved in 1-methylimidazole (15 ml); sodium azide (7.47 g; 115 mmol) and triethylamine hydrochloride (12.4 g 90 mmol) were added and the reaction mixture was heated for 12h at around 130°C. The reaction mixture was then cooled to room temperature and 104 ml of 1M NaOH and 104 ml of *tert*-butyl methyl ether was added. The phases were separated and to aqueous phase 7.2 g (104 mmol) NaNO₂ was added and then the solution was slowly added to 405 ml of 1M HCl (pH<1). Irbesartan hydrochloride precipitated from the mixture. The precipitate was separated and dried.
Yield: 20.9 g (94 %) HPLC Area %:96.49

### Process H

*N*-[(2'-cyanobiphenyl-4-yl)methyl]-1-pentanamidocyclopentanecarboxamide (10 g, 25.9 mmol), triethylamine hydrochloride (7.13 g; 51.8 mmol) and sodium azide (3.37 g, 51.8 mmol) were dissolved in 1-methylpyrrolidin-2-one (2 ml) and heated 12h at 130-140°C. Reaction mixture was then cooled to below 50°C, sodium hydroxide (36.7 ml, 0.24 M) and 37 ml of *tert*-butyl methyl ether were added and stirred for 30 min. To the aqueous phase 0.89 g of NaNO₂ was added. The solution was slowly poured into 26 ml 3.5 M. HCl Irbesartan hydrochloride precipitated from the mixture. The precipitate was separated and dried.
Yield: 6.04 g (82 %) HPLC Area %:95.5

The crude products obtained from examples 4 were recrystallized from mixture 2-butanone : 3M HCl (9:1;V:V)

### Example 5:

Tablets with the compositions listed in below Tables were prepared according to the wet granulation process as follows.

### 1) Preparation of granulates (1a)-(6a) and (1a')-(6a')

The respective solid components shown in table 1 below were homogenized in a high-shear mixer, and ethanol (e.g. ethanol 96 vol. % or absolute ethanol, diluted with 10 w/w% of purified water) was sprayed onto this mixture. The obtained granulate was dried in a drying chamber or a fluid bed dryer at a specified temperature. The obtained granulate was sieved and the drying loss was determined, using a Mettler Toledo HR73 moisture analyzer at 85°C for 20 minutes. The nature and amounts of the solid components and of the ethanol, the drying device, drying temperature and the drying loss of the sieved granulate are shown in Tables 1A and 1B.

**Table 1A**

| | Granulate | | | | | |
|---|---|---|---|---|---|---|
| | 1a | 2a | 3a | 4a | 5a | 6a |
| Components (g) | | | | | | |
| Irbesartan-HCl | 344.43 | 344.43 | 344.43 | 344.43 | 344.43 | 344.43 |
| Hydrochlorothiazide | - | - | 25 | 25 | 25 | 25 |
| Mannitol | 246.57 | 194.57 | 194.57 | 214.57 | 246.57 | 194.57 |
| Klucel EF²⁾ | 20 | 40 | 40 | 20 | 20 | 40 |
| LH-21³⁾ | 28 | 60 | 60 | 28 | 28 | 28 |
| Ethanol 96 vol. % | 60 | - | - | 60 | - | 60 |
| Absolute ethanol+purified water | - | 54+6 | 54+6 | - | 54+6 | - |
| Drying method⁴⁾ | FBD | FBD | DC | FBD | FBD | FBD |
| Drying temperature (°C)⁵⁾ | 45-52 | 43-47 | 35-40 | 45-50 | 44-51 | 45-51 |
| Drying loss of granulate (w/w %) | 2.60 | 2.89 | 3.20 | 2.76 | 2.97 | 3.02 |

**Table 1B**

| | Granulate | | | | | |
|---|---|---|---|---|---|---|
| | 1a' | 2a' | 3a' | 4a' | 5a' | 6a' |
| Components (g) | | | | | | |
| Irbesartan-HCl¹⁾ | 344.43 | 344.43 | 344.43 | 344.43 | 344.43 | 344.43 |
| Hydrochlorothiazide | - | - | 12.5 | 12.5 | 12.5 | 12.5 |
| Mannitol | 259.07 | 207.07 | 207.07 | 227.07 | 259.07 | 207.07 |
| Klucel EF²) | 20 | 40 | 40 | 20 | 20 | 40 |
| LH-21³⁾ | 28 | 60 | 60 | 28 | 28 | 28 |
| Ethanol 96 vol. % | 60 | - | - | 60 | - | 60 |
| Absolute ethanol+purified water | - | 54+6 | 54+6 | - | 54+6 | - |
| Drying method⁴⁾ | FBD | FBD | DC | FBD | FBD | FBD |
| Drying temperature (°C)⁵⁾ | 45-51 | 44-49 | 35-40 | 45-50 | 43-49 | 45-52 |
| Drying loss of granulate (w/w %) | 2.39 | 2.69 | 2.88 | 2.37 | 2.49 | 2.98 |

| | | | | | | |
|---|---|---|---|---|---|---|
| Ethanol 96 vol. % and absolute ethanol+purified water are used as a granulating liquid and evaporate during drying of the granulate. ¹⁾ as irbesartan-HCl.1.5 H₂O (344.43 g of irbesartan-HCl.1.5 H₂O correspond to 300 g free irbesartan) ²⁾ Klucel EF: commercially available hydroxypropylcellulose ³⁾ LH-21: commercially available low-substituted hydroxypropylcellulose ⁴⁾ DC: drying chamber FBD: fluid-bed dryer ⁵⁾ inlet air temperature | | | | | | |

### 2) Preparation of cmpression mixtures (1b)-(6b) and (1b')-(6b')

The components shown in Tables 2A and 2B were added to the respective granulates (1a)-(6a) obtained above and mixed in a high-shear mixer to obtain a compression mixture. The drying loss of the compression mixtures was determined, using a Mettler Toledo HR73 moisture analyzer at 85°C for 20 minutes. The amounts of of the added components as well as the drying loss of the compression mixtures are shown in Table 2.

**Table 2A**

| | Compression mixture | | | | | |
|---|---|---|---|---|---|---|
| | 1b | 2b | 3b | 4b | 5b | 6b |
| Granulate | 1a | 2a | 3a | 4a | 5a | 6a |
| Components (g) | | | | | | |
| Hydrochlorothiazide | 25 | 25 | - | - | - | - |
| LH-111) | 60 | 60 | 60 | 92 | 60 | 92 |
| Talc | 36 | 36 | 36 | 36 | 36 | 36 |
| Macrogol 6000 | 24 | 24 | 24 | 24 | 24 | 24 |
| Hydrogenated castor oil | 16 | 16 | 16 | 16 | 16 | 16 |
| Drying loss of compression mixture (w/w %) | 2.49 | 2.65 | 3.02 | 2.89 | 2.92 | 3.13 |

**Table 28**

| | Compression mixture | | | | | |
|---|---|---|---|---|---|---|
| | 1b' | 2b' | 3b' | 4b' | 5b' | 6b' |
| Granulate | 1a' | 2a' | 3a' | 4a' | 5a' | 6a' |
| Components (g) | | | | | | |
| Hydrochlorothiazide | 25 | 25 | - | - | - | - |
| LH-111) | 60 | 60 | 60 | 92 | 60 | 92 |
| Talc | 36 | 36 | 36 | 36 | 36 | 36 |
| Macrogol 6000 | 24 | 24 | 24 | 24 | 24 | 24 |
| Hydrogenated castor oil | 16 | 16 | 16 | 16 | 16 | 16 |
| Drying loss of compression mixture (w/w %) | 1.95 | 2.67 | 2.43 | 2.23 | 2.19 | 2.98 |

| | | | | | | |
|---|---|---|---|---|---|---|
| ¹⁾ LH-11: commercially available low-substituted hydroxypropylcellulase | | | | | | |

### 3) Preparation of compressed tablet cores

A part of the compression mixtures (1b)-(6b) was compressed into capsule shaped tablet cores (1ca)-(6ca) with theoretical weight of 800 mg, while the remaining compression mixtures (1b)-(6b) were compressed into oval shaped tablet cores (1cb)-(6cb). The compression mixtures (1b')-(6b') were compressed into capsule shaped tablet cores (1c')-(6c') with theoretical weight of 800 mg. The hardness of the compressed tablet cores and the disintegration time (in minutes) thereof in purified water at 37°C was measured according to Ph.Eur.. The results are shown in Tables 3A-3C.

**Table 3A**

| | Compressed tablet cores | | | | | |
|---|---|---|---|---|---|---|
| | 1ca | 2ca | 3ca | 4ca | 5ca | 6ca |
| Compression mixture | 1b | 2b | 3b | 4b | 5b | 6b |
| Weight (mg) | 800 | 800 | 800 | 800 | 800 | 800 |
| Hardness (N) | 168-190 | 183-205 | 172-201 | 156-176 | 166-198 | 152-173 |
| Disintegration time (min) | 2.5 | 3.5 | 3 | 2 | 4 | 1.5 |

**Table 3B**

| | Compressed tablet cores | | | | | |
|---|---|---|---|---|---|---|
| | 1cb | 2cb | 3cb | 4cb | 5cb | 6cb |
| Compression mixture | 1b | 2b | 3b | 4b | 5b | 6b |
| Weight (mg) | 400 | 400 | 400 | 400 | 400 | 400 |
| Hardness (N) | 154-171 | 139-168 | 134-172 | 152-180 | 142-163 | 133-148 |
| Disintegration time (min) | 3 | 6 | 5 | 6 | 4 | 3.5 |

**Table 3C**

| | Compressed tablet cores | | | | | |
|---|---|---|---|---|---|---|
| | 1c' | 2c' | 3c' | 4c' | 5c' | 6c' |
| Compression mixture | 1b' | 2b' | 3b' | 4b' | 5b' | 6b' |
| Weight (mg) | 800 | 800 | 800 | 800 | 800 | 800 |
| Hardness (N) | 181-212 | 187-213 | 171-192 | 165-179 | 162-196 | 155-174 |
| Disintegration time (min) | 2.5 | 3.5-4 | 2.5 | 2.5 | 3.5 | 2 |

In addition, the compressed tablet cores (1ca)-(6ca), (1cb)-(6cb) and (1c')-(6c') were coated in an automatic caoting pan with water-based film coating suspension of a ready-to-make mixture, commercially available as Opadry II HP. The theoretical weight of the coated tablets containing tablet cores (1ca)-(6ca) and (1c')-(6c') was 824 mg. The theoretical weight of the coated tablets containing tablet cores (1cb)-(6cb) was 412 mg. The resulting coating thickness is appropriate to hide the unpleasant taste of irbesartan experienced when this is dissolved into saliva.

### Example 6

### Preparations 1-14

Tablets were prepared according to the wet granulation process as follows.

### 1) Preparation of granulates (1a) - (14a)

The respective solid components shown in Table 4 below were homogenized in a high-shear mixer, and ethanol was sprayed over this mixture. The obtained granulate was dried in a fluid-bed dryer at a specified temperature. The obtained granulate was sieved and the drying loss was measured using a Mettler Toledo HR73 halogen moisture analyzer at 85°C for 20 minutes. The nature and amounts of the solid components and of the ethanol, drying temperature and the drying loss of the sieved granulate are shown in Table 4.

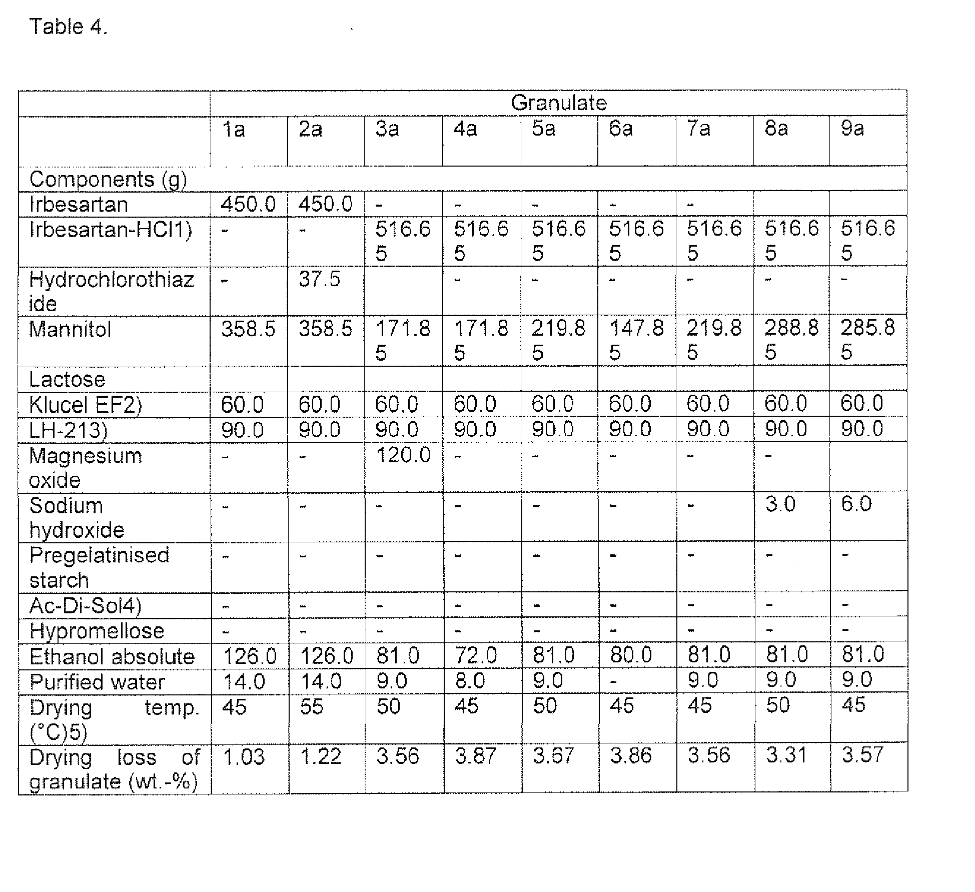

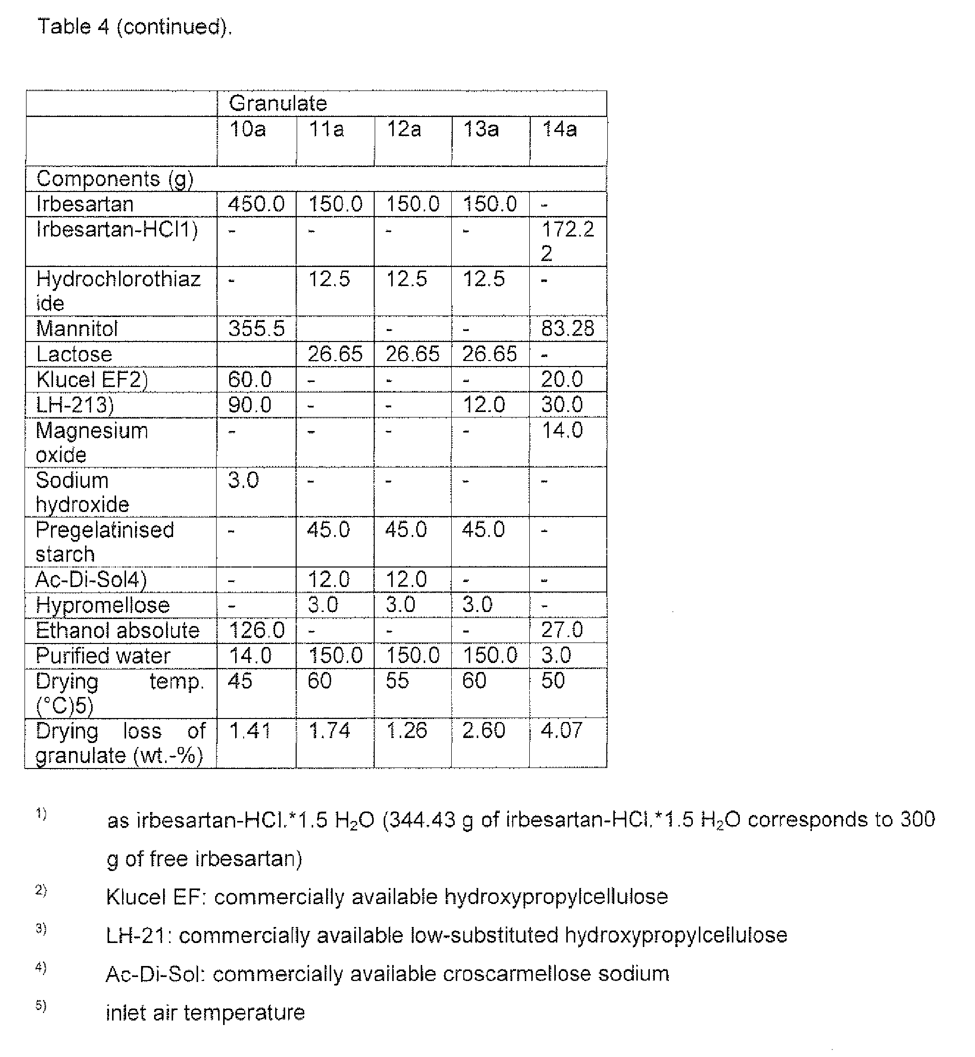

### 2) Preparation of compression mixtures (1b)-(14b)

The components shown in Table 5 below were added to the respective granulates (1a)-(14a) obtained above and mixed in a high-shear mixer to obtain a compression mixture. The drying loss was measured using a Mettler Toledo HR73 halogen moisture analyzer at 85°C for 20 minutes. The amounts of the added components as well as the drying loss of the compression mixtures are shown in Table 5.

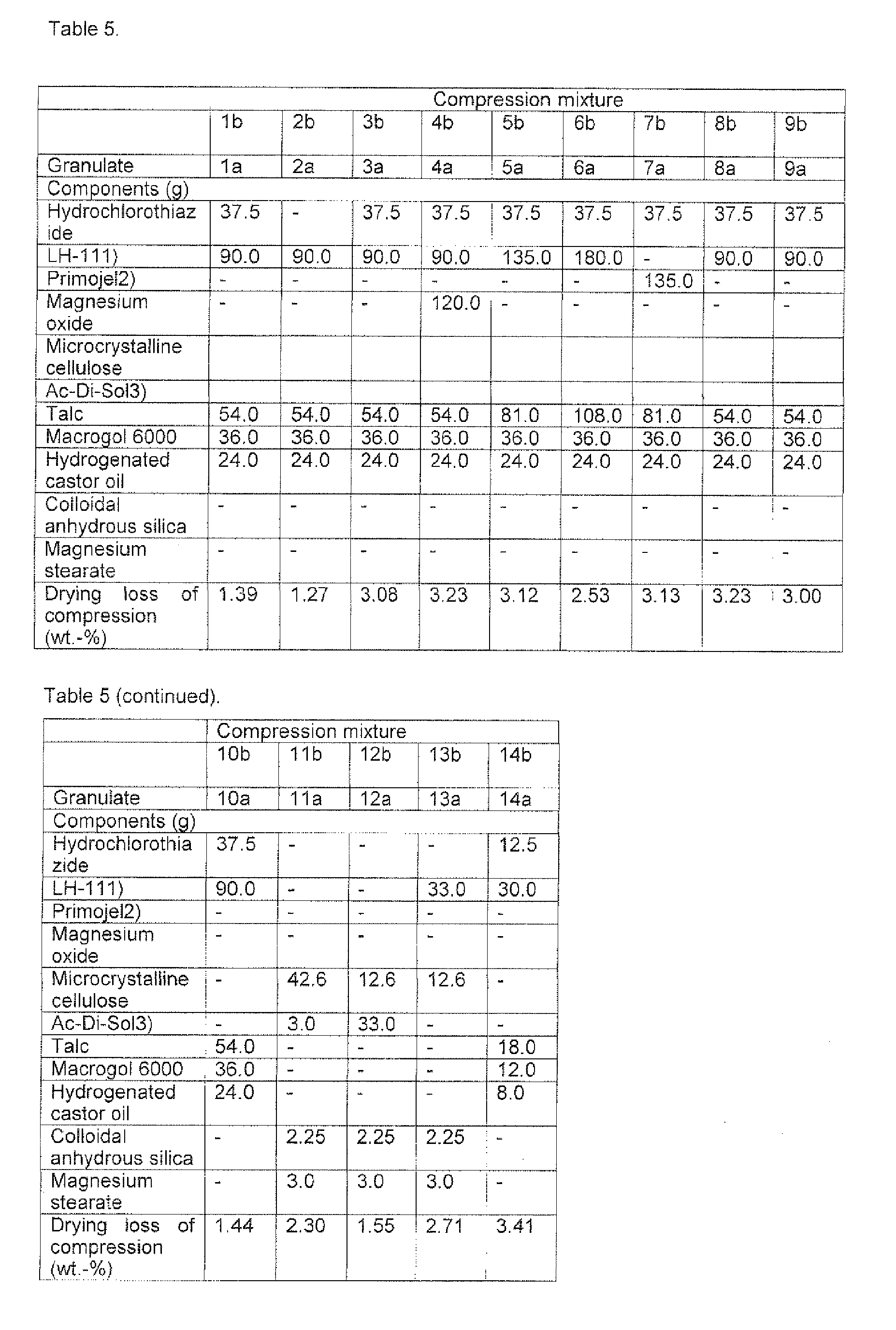

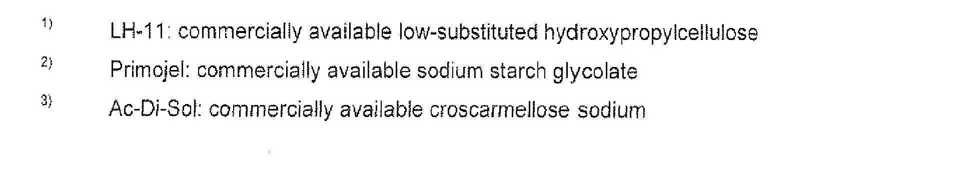

### 3) Preparation of compressed tablet cores (1c)-(14c)

The compression mixtures (1b)-(14b) were compressed into oval tablet cores (1c)-(14c) with theoretical weight of 800 mg or 600 mg. The hardness of the compressed tablet cores and the disintegration time thereof in purified water at 37°C was measured according to Ph. Eur. The results are shown in Table 6.

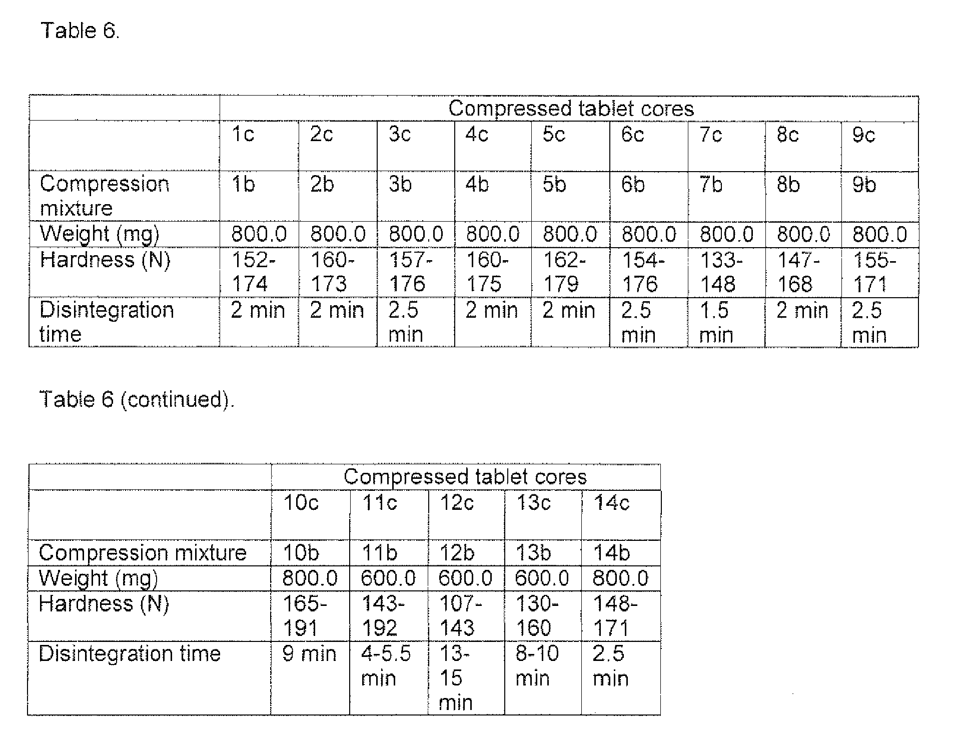

In addition, the compressed tablet cores were coated in an aoutomatic coating pan with water-based film coating suspension of a ready-to-make mixture, commerically available as Opadry II HP. The theoretical weight of the coated tablets containing cores (1c)-(10c) and (14c) was 824 mg, and of the cores (11c)-(13c) 620 mg. The resulting coating thickness is appropriate to hide unpleasant taste of irbesartan experienced when thus is dissolved into the saliva.

### Example 7

### Preparations 15-16

Tablets were prepared according to the dry granulaton of the granulate containing irbesartan as comprised in Table 7.

### 1) Preparation of granulates (15a) - (16a)

The respective solid components shown in Table 7 below were homogenized in a mixer and dry granulated on a roller compactor Chilsonator with integrated mill IR220/L1A (manufactured by Fitzpatrick).

**Table 7.**

| | Granulate | |
|---|---|---|
| | 15a | 16a |
| Components (g) | | |
| Irbesartan-HCl1) | 1722.1 5 | 1722.15 |
| Mannitol | 732.85 | 832.85 |
| Klucel EF2) | 200.0 | 200.0 |
| LH-213) | 300.0 | 300.0 |
| Talc | 90.0 | - |
| Magnesium oxide | - | 140.0 |

| | | |
|---|---|---|
| ¹⁾ as irbesartan-HCl.*1.5 H₂O (344.43 g of irbesartan-HCl.*1.5 H₂O corresponds to 300 g of free irbesartan) ²⁾ Klucel EF: commercially available hydroxypropylcellulose ³⁾ LH-21: commercially available low-substituted hydroxypropylcellulose | | |

### 2) Preparation of compression mixtures (15b)-(16b)

The components shown in Table 8 below were added to the respective granulates (1 5a)-(16a) obtained above and mixed in a high-shear mixer to obtain a compression mixture. The drying loss was measured using a Mettler Toledo HR73 halogen moisture analyzer at 85°C for 20 minutes. The amounts of the added components as well as the drying loss of the compression mixtures are shown in Table 8.

**Table 8.**

| | Compression mixture | |
|---|---|---|
| | 15b | 16b |
| Granulate | 15a | 10a |
| Components (g) | | |
| Hydrochlorothiazide | 125.0 | - |
| Primojel1) | 450.0 | |
| LH-112) | - | 300.0 |
| Talc | 180.0 | 180.0 |
| Macrogol 6000 | 120.0 | 120.0 |
| Hydrogenated castor oil | 80.0 | 80.0 |
| Drying loss of compression (wt.-%) | 3.45 | 3.78 |

| | | |
|---|---|---|
| ¹⁾ Primojel: commercially available sodium starch glycolate ²⁾ LH-11: commercially available low-substituted hydroxypropylcellulose ³⁾ inlet air temperature | | |

### 3) Preparation of compressed tablet cores (15c)-(16c)

The compression mixtures (15b)-(16b) were compressed into oval tablet cores (15c)-(16c) with theoretical weight of 800 mg. The disintegration time of the tablet cores (15c) was 5-5.5 minutes.

In addition, the compressed tablet cores were optionally coated in an automatic coating pan with water-based film coating suspension of a ready-to-make mixture, commercially available as Opadry II HP. The theoretical weight of the coated tablets contatining cores was 824 mg.

### Example 8

### Samples 17-18

Tablets were prepared according to the wet granulation process as follows in order to prepare bilayer tablets.

### ¹⁾ Preparation of granulates (17a) - (18a)

The respective solid components shown in Table 9 below were homogenized in a high-shear mixer, and ethanol was sprayed over this mixture. The obtained granulate was dried in a fluid-bed dryer at a specified temperature. The obtained granulate was sieved and the drying loss was measured using a Mettler Toledo HR73 halogen moisture analyzer at 85°C for 20 minutes. The nature and amounts of of the solid components and of the ethanol, the drying device, drying temperature and the drying loss of the sieved granulate are shown in Table 9.

**Table 9.**

| | Granulate | |
|---|---|---|
| | 17a | 18a |
| Components (g) | | |
| Irbesartan-HCl1) | 861.075 | 861.075 |
| Mannitol | 206.425 | 216.425 |
| Klucel EF2) | 100.0 | 100.0 |
| LH-213) | 150.0 | 150.0 |
| Magnesium oxide | - | 70.0 |
| Ethanol absolute | 150.0 | 150.0 |
| Purified water | - | - |
| Drying method/loss | | |
| Drying temp. (°C)4) | 45 | 50 |
| Drying loss of granulate (wt.-%) | 3.96 | 3.10 |

| | | |
|---|---|---|
| ¹⁾ as irbesartan-HCl.*1.5 H₂O (344.43 g of irbesartan-HCl.*1.5 H₂O corresponds to 300 g of free irbesartan) ²⁾ Klucel EF: commercially available hydroxypropylcellulose ³⁾ LH-21: commercially available low-substituted hydroxypropylcellulose | | |

### 2) Preparation of compression mixtures (17b)-(18b) and (17b')-(18b')

The components shown in Table 10A below were added to the respective gradates (17a)-(16a) obtained above and mixed in a high-shear mixer to obtain a compression mixture, containing irbesartan. The amounts of the added components are shown in Table 10A.

**Table 10A.**

| | Compression mixture | |
|---|---|---|
| | 17b | 18b |
| Granulate | 17a | 18a |
| Components (g) | | |
| Primojel¹⁾ | 150.0 | - |
| LH-11²⁾ | - | 100.0 |
| Talc | 90.0 | 60.0 |
| Macrogol 6000 | 40.0 | 40.0 |
| Hydrogenated castor oil | 27.5 | 27.5 |

| | | |
|---|---|---|
| ¹⁾ Primojel: commercially available sodium starch glycolate ²⁾ LH-11: commercially available low-substituted hydroxypropylcellulose | | |

The components shown in Table 10B below were mixed in a high-shear mixer to obtain a compression mixture, containing hydochlorothiazide. The amounts of the added components are shown in Table 10B.

**Table 10B.**

| | Compression mixture | |
|---|---|---|
| | 17b' | 18b' |
| Components (g) | | |
| Hydrochlorothiaz ide | 62.5 | 62.5 |
| Mannitol | 165.0 | 200.0 |
| Primojel¹⁾ | 75.0 | - |
| LH-11²⁾ | - | 50.0 |
| Talc | 45.0 | 30.0 |
| Macrogol 6000 | 15.0 | 40.0 |
| Hydrogenated castor oil | 12.5 | 27.5 |

| | | |
|---|---|---|
| ¹⁾ Primojel: commercially available sodium starch glycolate ²⁾ LH-11: commercially available low-substituted hydroxypropylcellulose | | |

### 3) Preparation of tablet cores (17c)-(18c)

The compression mixtures (17b) and (17b') and (18b) and (18b') were compressed into oval tablet cores (17c)-(18c) with theoretical weight of 800 mg, wherein theoretical weight of the first tablet layer is 650 mg and of the second layer is 150 mg.

In addition, the compressed tablet cores were optionally coated in an automatic coating pan with water-based film coating suspension of a ready-to-make mixture, commerically available as Opadry II HP. The theoretical weight of the coated tablets contatining cores was 824 mg.

## Claims

1. A process for the preparation of N-[(2'-cyanobiphenyl-4-yl)methyl]-1-pentanamidocyclopentanecarboxamide of formula (IIIa), or a pharmaceutical acceptable salt thereof, comprising the step of *N*-alkylation of a primary amide group of 1-pentanamidocyclopentanecarboxamide of formula (V) with a 4'-substituted methyl biphenyl-2-carbonitrile of formula (IVa) as shown below: 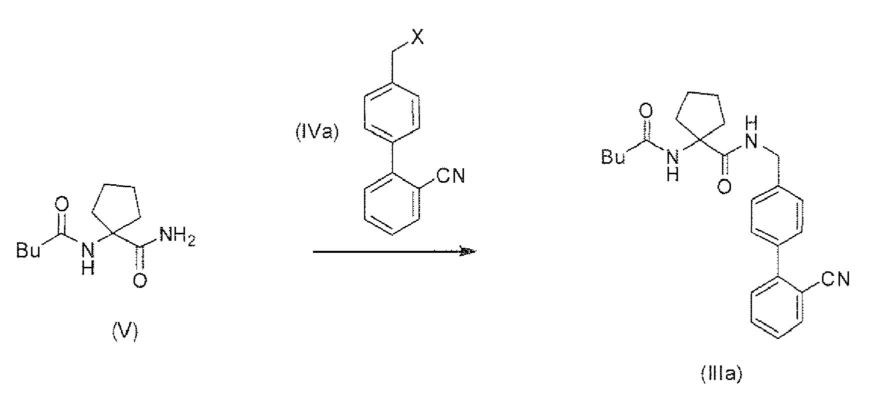 wherein X is a halogen atom or a leaving group, such as for example Cl, Br, I, tosylate, mesylate or triflate.

2. A process for the preparation of *N*-[1-({[2'-(1*H*-tetrazol-1-yl)biphenyl-4-yl]methylamino}methyl)cyclopentyl]pentanamide of formula (IIIb), or a pharmaceutical acceptable salt thereof, comprising the step of *N-*alkylation of primary amide group of 1-pentanamidocyclopentanecarboxamide of formula (V) with 1-(4'-substituted methyl biphenyl-2-yl)-1H-tetrazole of formula (IVb) as shown below: 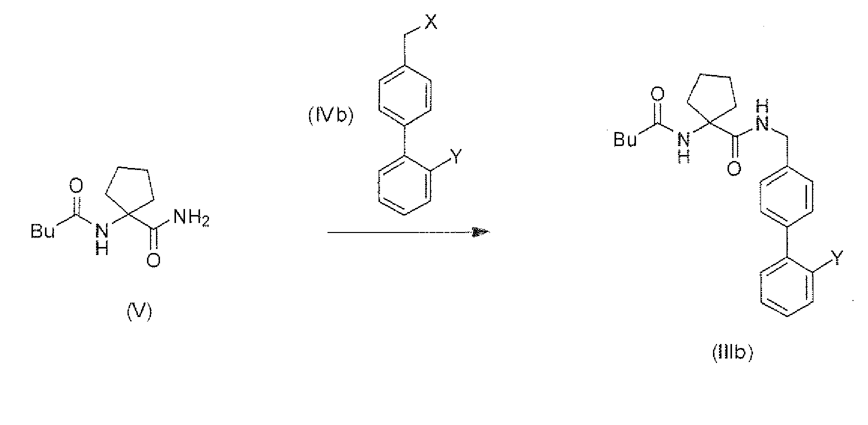 wherein Y represents a protected or unprotected tetrazole group and wherein X is a halogen atom or a leaving group, such as for example Cl, Br, I, tosylate, mesylate or triflate.

3. The process according to any of claims 1 to 2 wherein the *N*-alhylation of the primary amide group of 1-pentanamidocyclopentanecarboxamide of formula (V) is performed in an organic solvent or solvent mixture comprising at least one organic solvent in the presence of a base, preferably a metal hydroxide.

4. The process according to claim 3 wherein the base is KOH.

5. The process according to any of claims 3, or 4, wherein the *N*-alkylation of the primary amide group of 1-pentanamidocyclopentanecarboxamide of formula (V) is conducted in acetonitrile.

6. The process according to any of claims 1 to 5 wherein the compound of formula (IIIa) or (IIIb) is purified to a degree of purity of at least 97%, preferably of at least 98% and more preferably of at least 99%.

7. The process according to any of claims 1 to 6 wherein the compound of formula (IIIa) or (IIIb) is purified by recrystallization in an organic solvent or any mixtures thereof, preferably in acetone.

8. A process for the preparation of Irbesartan or a pharmaceutically acceptable salt thereof,
(ia) either comprising as a process step the process of preparing compound (IIIa) as specified in any one of claims 1, 3, 4, 5, 6 and 7,
or alternatively
(ib) comprising as a process step the process of preparing compound (IIIb) as specified in any one of claims 2, 3, 4, 5, 6, and 7;
and/or
(ii) comprising the step of converting 4'-[(2-butyl-4-oxo-1,3-diazaspiro[4.4]non-1-en-3-yl)methyl]biphenyl-2-carbonitrile of formula (IIa) to obtain Irbesartan (I) as shown below: 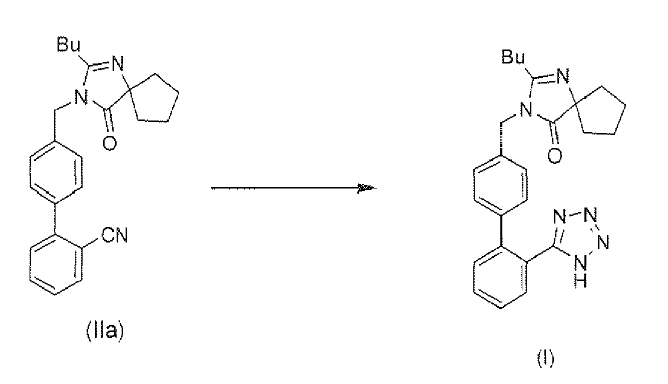
wherein this conversion is conducted with sodium azide in a basic organic solvent, preferably in 1- methylimidazole.

9. The process according to claim 8, wherein the pharmaceutically acceptable salt of Irbesartan is the hydrochloride.

10. The process for the preparation of lrbesartan or a pharmaceutical acceptable salt thereof according to any one of claims 8 and 9, comprising the steps of:
a) the process of preparing compound (IIIa) as specified in any one of claims 1, 3, 4, 5, 6 and 7;
b) cyclization of the pentanamidocyclopentanecarboxamide moiety of N-[(2'-cyanobiphenyl-4-yl)methyl]-1-pentanamidocyclopentanecarboxamide of formula (IIIa) to obtain the 4'-[(2-butyl-4-oxo-1,3-diazaspiro[4.4]non-1-en-3-yl)methyl]biphenyl-2-carbonitrile of formula (IIa) as shown below: 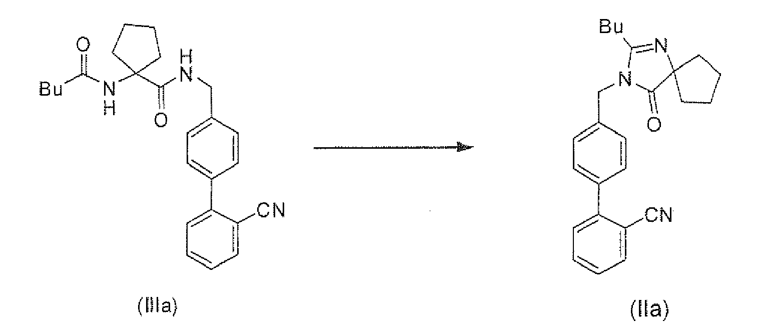
c) converting the carbonitrile moiety of 4'-[(2-butyl-4-oxo-1,3-diazaspiro[4.4]non-1-en-3-yl)methyl]biphenyl-2-carbonitrile of formula (IIa), preferably with sodium azide, into a tetrazolyl moiety to obtain Irbesartan (I) as shown below: 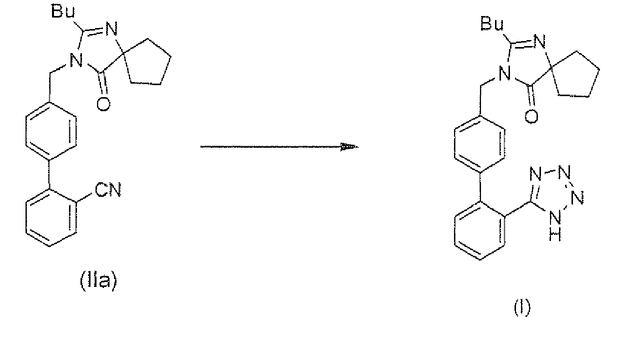
d) optionally converting Irbesartan into one of its pharmaceutically acceptable salts, preferably Irbesartan-HCl (la), as shown below:

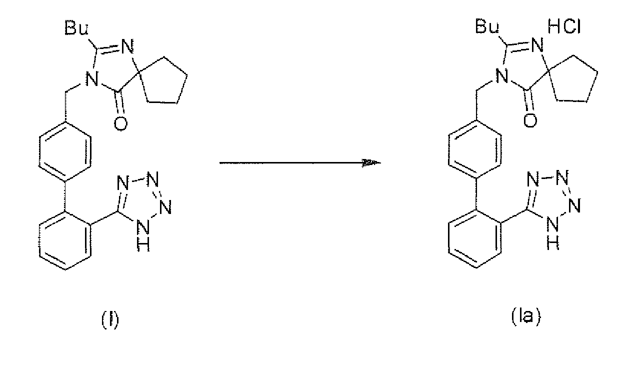

11. The process according to claim 10 wherein the conversion of 4'-[(2-butyl-4-oxo-1,3-diazaspiro[4.4]non-1-en-3-yl)methyl]biphenyl-2-carbonitrile of formula (IIa) to obtain lrbesartan (I) is conducted with sodium azide in a basic organic solvent, preferably in 1-methylimidazole.

12. The process according to claim 8, variant (ii), or claim 10 or 11, wherein the conversion of compound (IIa) to Irbesartan is catalyzed by the addition of an acid, preferably acetic acid, hydrochloric acid or triethylamine hydrochloride.

13. A process for the preparation of a pharmaceutical composition comprising the steps of preparing Irbesartan or a pharmaceutically acceptable salt thereof by a process according to any of the preceding claims 8 to 12 followed by mixing a therapeutically effective amount thereof with one or more pharmaceutically acceptable excipient(s) and optionally with one or more other active substance(s).

14. Process according to claim 13, wherein the pharmaceutical composition is for the treatment of hypertension.
